# EUROPEAN PATENT APPLICATION

(11) **EP 2 578 167 A1**
(43) Date of publication of application: **10.04.2013**
(21) Application number: 12187293.1
(22) Date of filing: 04.10.2012
(51) Int. Cl.: A61B 17/02

(54) **Surgical retractor**

(30) Priority: 04.10.2011 US 201161543172 P
(71) Applicant: Cook Medical Technologies LLC, Bloomington, IN 47404 (US)
(72) Inventor: Jaworek, Jacqueline A., Bloomington, IN Indiana 47403 (US); Kamel, Amro, Bloomington, IN Indiana 47403 (US); Bosel, Christopher D., Bloomington, IN Indiana 47404 (US); Melsheimer, Jeffry S., Springville, IN Indiana 47462 (US); Burton, Christopher W., New Whiteland, IN Indiana 46184 (US); Ryan, Chelsey, Bloomington, IN Indiana 47404 (US); Whetham, Daniel K., Bloomington, IN Indiana 47404 (US); Fercik-Grant, Carrie, Ellettsville, IN Indiana 47429 (US); Theobald, Elizabeth A., Bloomington, IN Indiana 47403 (US); Wittenberg, Ellen, Westlake, OH Ohio 44145 (US)
(74) Representative: Garratt, Peter Douglas

(57) **Abstract**

The exemplary embodiments illustrated provide the discovery of apparatuses and methods of use of surgical retractors that solve the problems presently posed through use of the Trendelenberg position, present graspers, and present retractors. The embodiments are conformable into a sufficiently small configuration for introduction and positioning within a surgical site, and thereafter, are configurable into a larger, more rigid structure to sufficiently retain tissues and organs from the surgical site.

## Description

### TECHNICAL FIELD

The present invention relates to medical devices and more specifically, to devices used in conjunction with minimally invasive surgical procedures.

### BACKGROUND

Laparoscopic surgical procedures generally involve inflating a bodily cavity with a gas, such as the abdomen, to provide better access to the surgical site. Such gasses may include carbon dioxide. After the surgical site is insufflated, the bodily cavity is punctured generally using a trocar device. For example, when performing laparoscopic surgery in the abdominal area, the trocar device is utilized to puncture the peritoneum, and an access port, such as a cannula, is inserted through the puncture to provide an access means through the abdominal wall for the introduction of surgical instrumentation.

Often times, a number of incisions are made and access ports are inserted so as to provide sufficient access means to the surgical site for a number of surgical instruments necessary to complete the surgical procedure. For example, a surgeon may utilize one access port through which to pass an endoscope for direct visualization of the surgical site, and one or more additional access ports for the introduction of surgical instruments, such as forceps, graspers, staples, scissors, and/or other surgical instruments.

Laparoscopic surgery has many advantages over traditional open surgery in that it generally takes less time to complete, the patient is likely to experience less severe post-operative pain, and the incisions leave less noticeable scarring as compared to open surgery. Additionally, hospital recovery time and cost are generally reduced.

Despite the benefits of laparoscopic surgery, such surgery is often difficult to perform, especially in the case of hand-assisted laparoscopic surgery wherein a surgeon places one of her hands into the surgical site through a traditional scalpel incision along with any number of surgical instruments through one or more access ports. One difficulty in performing laparoscopic surgery is due to the small working space and the need to push back and protect delicate tissues and organs from the surgical site. While insufflation gas expands the surgical site allowing a surgeon to view the site, such gas is generally unable to strategically move and manipulate internal tissues and organs to provide a clear pathway to the surgical objective. In the case of abdominal surgery, the bowel generally moves toward the surgical site, and accidently perforating it could cause severe injury or death to a patient. In an effort to keep the bowel and other tissues and organs away from the surgical site, surgeons may position the patient in the Trendelenberg position and/or use present graspers or present retractors. But none provide effective solutions.

The Trendelenberg position, wherein a patient is tilted backwards such that her head is downward to her feet, is used to encourage gravity to move organs from the surgical site. However, a patient subjected to the Trendelenberg position for sufficiently long time durations may suffer breathing difficulties and increased blood pressure at the head and at other tissues and organs. Such side effects of the Trendelenberg position may present significant risk to a patient, including death.

Present graspers, for example, are generally overly rigid such that they are difficult to navigate and direct to the area needed, and once there, they are difficult to maintain in position. Moreover, their rigidity may present a hazard to soft tissues and organs by accidental perforation. Present retractors, for example, are generally cumbersome, awkwardly shaped, and too narrow to effectively shield and hold back multiple tissues and organs from the surgical site.

### BRIEF SUMMARY

In a first aspect, a method of retaining material from a surgical site is provided, including providing a surgical retractor including: a frame; and a membrane, wherein a perimeter of the membrane is at least partially bounded by the frame; wherein the surgical retractor is configurable into a compressed configuration and an uncompressed-expanded configuration, wherein when in the compressed configuration, the surgical retractor includes a width less than the width of the surgical retractor when in the uncompressed-expanded configuration; wherein when in the uncompressed-expanded configuration, the membrane is sufficiently taut; inserting the surgical retractor in the compressed configuration between a surgical site and an area to be retained; and configuring the surgical retractor into the uncompressed-expanded configuration such that the surgical retractor forms a barrier between the surgical site and the area to be retained.

In a second aspect, a method of retaining material from a surgical site is provided, including providing a surgical retractor including: a frame; a membrane, wherein a perimeter of the membrane is at least partially bounded by the frame; and means for configuring the surgical retractor between a compressed configuration and an uncompressed-expanded configuration, wherein the surgical retractor comprises a width when in the compressed configuration that is less than the width of the surgical retractor when in the uncompressed-expanded configuration; wherein when in the uncompressed-expanded configuration, the membrane is sufficiently taut; inserting the surgical retractor in the compressed configuration between a surgical site and an area to be retained; and configuring the surgical retractor into the uncompressed-expanded configuration such that the surgical retractor forms a barrier between the surgical site and the area to be retained.

In a third aspect, a surgical retractor is provided, including a frame; and a membrane, wherein a perimeter of the membrane is at least partially bounded by the frame; means for configuring the surgical retractor between a compressed configuration and an uncompressed-expanded configuration, wherein the surgical retractor includes a width when in the compressed configuration that is less than the width of the surgical retractor when in the uncompressed-expanded configuration; and wherein when in an uncompressed-expanded configuration, the membrane is sufficiently taut.

### BRIEF DESCRIPTION OF THE SEVERAL VIEWS OF THE DRAWINGS

The embodiments will be further described in connection with the attached drawing figures. It is intended that the drawings included as a part of this specification be illustrative of the exemplary embodiments and should in no way be considered as a limitation on the scope of the invention. Indeed, the present disclosure specifically contemplates other embodiments not illustrated but intended to be included in the claims. Moreover, it is understood that the figures are not necessarily drawn to scale.

Fig. 1A illustrates a side view of a surgical retractor in a deflated, compressed configuration;

Fig. 1B illustrates a side view of the surgical retractor illustrated in Fig. 1A in an uncompressed-expanded configuration;

Fig. 1C illustrates a cross-sectional view of the surgical retractor illustrated in Fig. 1B in use;

Fig. 2A illustrates a side view of a surgical retractor in a compressed configuration;

Fig. 2B illustrates a cross-sectional view of the surgical retractor illustrated in Fig. 2A in a partially expanded configuration;

Fig. 2C illustrates a cross-sectional view of the surgical retractor illustrated in Fig. 2A in an uncompressed-expanded configuration;

Fig. 2D illustrates an alternative cross-sectional view of the surgical retractor illustrated in Fig. 2A in an uncompressed-expanded configuration;

Fig. 3A illustrates a side view of a surgical retractor in a compressed configuration;

Fig. 3B illustrates a side view of the surgical retractor illustrated in Fig. 3A in an uncompressed-expanded configuration;

Fig. 4A illustrates a cross-sectional view of a surgical retractor in a compressed configuration;

Fig. 4B illustrates a cross-sectional view of the surgical retractor illustrated in Fig. 4A in an uncompressed-expanded configuration;

Fig. 5A illustrates a cross-sectional view of a surgical retractor in a compressed configuration;

Fig. 5B illustrates a cross-sectional view of the surgical retractor illustrated in Fig. 5A in an uncompressed-expanded configuration; and

Fig. 6 illustrates a method for deploying a retractor, including but not limited to, those illustrated in Figs. 1A-5B.

### DETAILED DESCRIPTION OF PRESENTLY PREFERRED EMBODIMENTS

The exemplary embodiments illustrated provide the discovery of apparatuses and methods of use of surgical retractors that solve the problems presently posed through use of the Trendelenberg position, present graspers, and present retractors. The embodiments are conformable into a sufficiently small configuration for introduction and positioning within a surgical site, and thereafter, are configurable into a larger, more rigid structure to sufficiently retain tissues and organs from entering the surgical site.

The present invention is not limited to those embodiments described herein, but rather, the disclosure includes all equivalents including those of different shapes, sizes, and configurations, including but not limited to, other retractors made from other materials. The devices, apparatuses, and methods can be used in any field benefiting from a retractor. Additionally, the devices and methods are not limited to being used with human beings, others are contemplated, including but not limited to, animals.

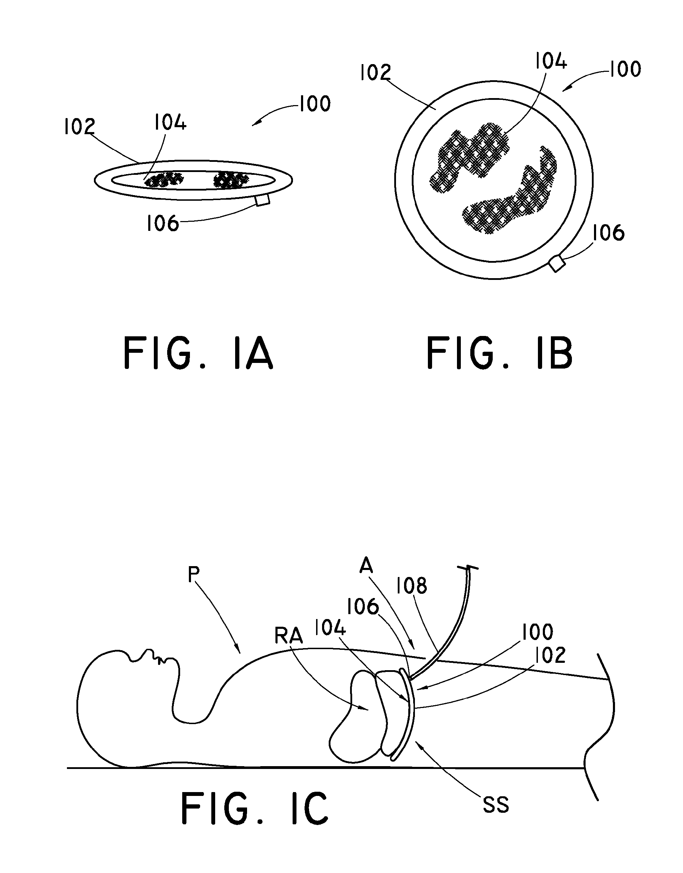

A more detailed description of the embodiments will now be given with reference to Figs. 1A-6. Throughout the disclosure, like reference numerals and letters refer to like elements. The present disclosure is not limited to the embodiments illustrated; to the contrary, the present disclosure specifically contemplates other embodiments not illustrated but intended to be included in the claims.

Fig. 1A illustrates a side view of surgical retractor 100 in a deflated, compressed configuration, Fig. 1B illustrates a side view of surgical retractor 100 in an uncompressed-expanded configuration, and Fig. 1C illustrates a cross-sectional view of surgical retractor 100 in use.

Retractor 100 is directed to hold back tissues and organs from a surgical site, including but not limited to, the bowel located in abdomen A (as illustrated in Fig. 1C), although retractor 100 can be used in other surgical and non-surgical applications. Retractor 100 is capable of assuming a compressed state (as illustrated in Fig. 1A), an uncompressed-expanded state (as illustrated in Fig. 1B), and a partially expanded state (not illustrated).

Retractor 100 is easy to deploy and position within an area of patient P, especially when used in conjunction with laparoscopic surgery and hand-assisted laparoscopic surgery. When configured in a compressed state (as illustrated in Fig. 1A), retractor 100 assumes a low profile making it easy to insert and position within patient P, such as that illustrated in Fig. 1C.

Referring to Figs. 1A-1C, retractor 100 includes frame 102, membrane 104, and inflation port 106. After being positioned, a fluid is directed through inflation tube 108 coupled to inflation port 106 thereby causing frame 102 to fill with the fluid. As frame 102 fills with the fluid, it expands into a circular or ovoid shape (or other desired configuration) having disposed within its perimeter membrane 104 and contouring to the specific anatomical features of patient P. Accordingly, membrane 104 becomes sufficiently taut to provide a barrier to effectively retain organs and tissues from a surgical site. Frame 102, when filled with a fluid, provides sufficient rigidity such that retractor 100 will not be pushed from the area in which it is located.

Membrane 104 provides a flexible surface against which delicate tissues and organs may contact without damaging such tissues and provides a barrier surface to prevent such tissues and organs from migrating from retracted area RA and into surgical site SS. Accordingly, unwanted tissues and organs are kept separate from surgical site SS.

To provide additional space about the surgical site, inflation tube 108 may be decoupled from inflation port 106. Accordingly, inflation port 106 is preferably equipped with a one-way valve such that the fluid will not leak therefrom if inflation tube 108 is decoupled from inflation port 106.

When retractor 100 is no longer needed, frame 102 may be punctured causing the fluid to release from frame 102 and thereby deflate and compress retractor 100 rapidly (or as slowly as desired by altering the size of the puncture into frame 102). Alternatively, frame 102 may be deflated by coupling inflation tube 108 to inflation port 106 and permitting the fluid to be removed therethrough. After deflation or partial deflation, retractor 100 can be removed from surgical site SS.

The fluid can be any biocompatible fluid, including but not limited to a liquid or gas. For example, the fluid preferably comprises carbon dioxide or saline, but other fluids are contemplated, including but not limited to, water. The fluid may be suctioned from the patient if needed or desired, such as in the case of when frame 102 is punctured to thereby allow the fluid to escape therefrom.

Retractor 100 is preferably sized so that it may be used in conjunction with laparoscopic surgery. It is contemplated that retractor 100 may be manufactured having a variety of sizes, such as small, medium, and large, so as to accommodate a variety of anatomical structures and locations. Other configurations, sizes, and dimensions are contemplated depending upon the surgical site in which retractor 100 may be deployed.

Frame 102 is preferably made from a high-pressure, non-compliant, fluid impermeable material, including but not limited to, nylon, polyethylene terephthalate ("PET"), silicone, polyesters, and polyurethanes.

Membrane 104 is preferably made from a deformable material, including but not limited to, nylon, rubber, silicone, and polyethylene, although other materials are contemplated, including but not limited to, plastic, fabric, woven materials, and polymers. Membrane 104 is preferably about 1- 5 mm thick, although other dimensions are contemplated depending upon the surgical site in which retractor 100 is to be deployed. It is contemplated that membrane 104 be configured sufficiently thin and flexible such that it is puncturable in the event the retained organs or tissues need to be accessed from the surgical site.

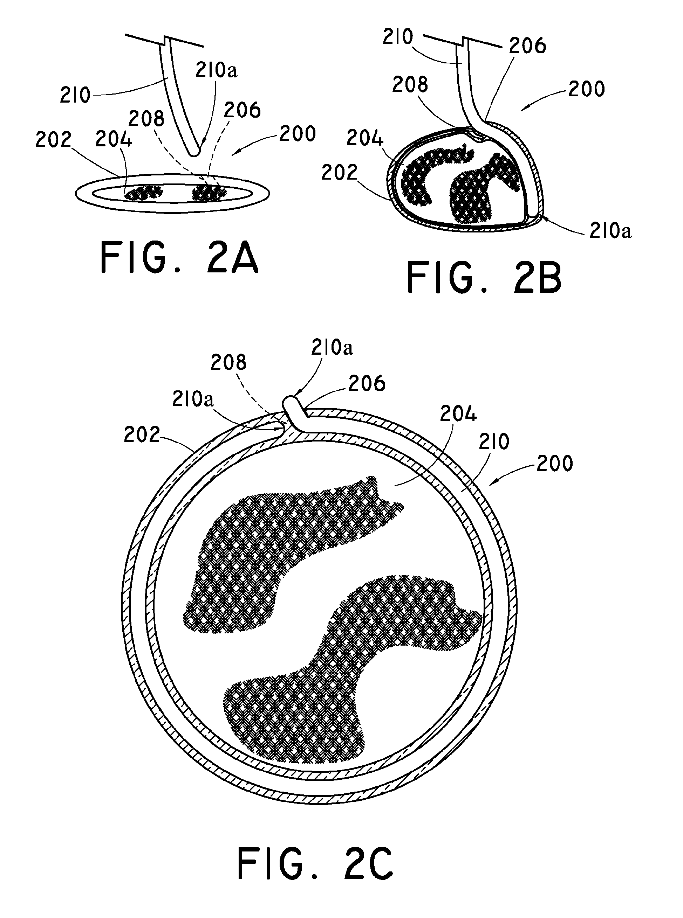

Fig. 2A illustrates side view of surgical retractor 200 in a compressed configuration, Fig. 2B illustrates a cross-sectional view of surgical retractor 200 in a partially expanded configuration, and Fig. 2C illustrates a cross-sectional view of surgical retractor 200 in an uncompressed-expanded configuration. Referring to Figs. 2A-2C, retractor 200 includes frame 202, membrane 204, wire opening port 206, wire stop portion 208, and wire 210 having atraumatic ends 210a.

Retractor 200 is directed to hold back tissues and organs from a surgical site, including but not limited to, the bowel located in the abdomen, although retractor 200 can be used in other surgical and non-surgical applications. Retractor 200 is capable of assuming a compressed state (as illustrated in Fig. 2A), a partially expanded state (as illustrated in Fig. 2B), and an uncompressed-expanded state (as illustrated in Fig. 2C).

Retractor 200 is easy to deploy and position within an area of a patient, especially when used in conjunction with laparoscopic surgery and hand-assisted laparoscopic surgery. When configured in a compressed state (as illustrated in Fig. 2A), retractor 200 assumes a low profile making it easy to insert and position within a patient.

After being positioned, wire 210 is directed into wire opening port 206 of frame 202, thereby causing frame 202 to stiffen and assume a partially uncompressed state (as illustrated in Fig. 2B). As wire 210 is directed further into and around the interior lumen within frame 202, frame 202 continues to expand into a circular or ovoid shape (or other desired configuration) having disposed within its perimeter membrane 204 and contouring to the specific anatomical features of the patient. Wire 210 is stopped from traveling any farther within lumen of frame 202 when it encounters stopping point 208. Because wire 210 has atraumatic ends 210a it will not puncture frame 202 or harm the patient when being introduced or removed from frame 202.

Membrane 204 becomes sufficiently taut to provide a barrier to effectively retain organs and tissues from a surgical site. Frame 202, having wire 210 disposed therein, provides sufficient rigidity such that retractor 200 will not be pushed from the area in which it is located.

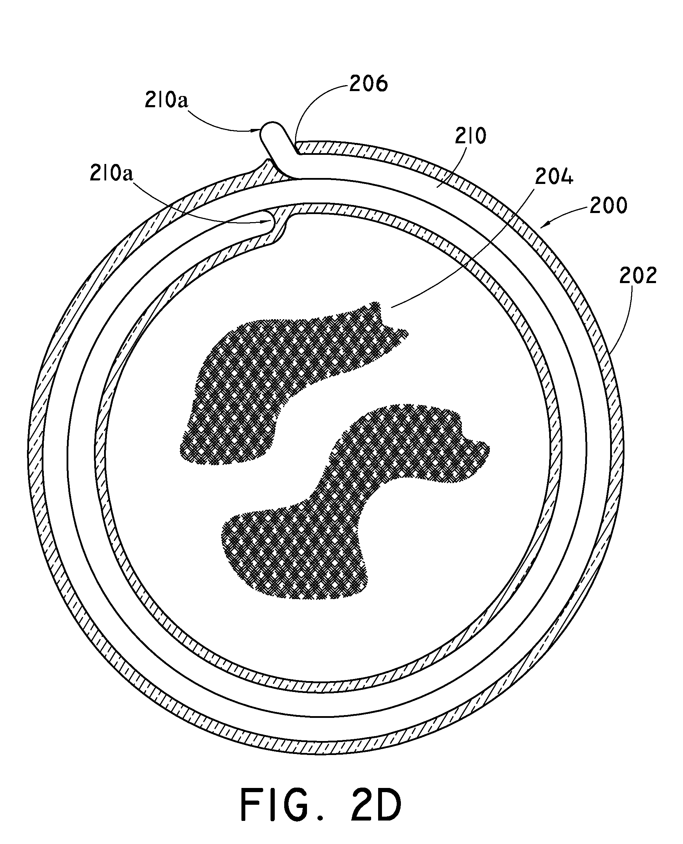

Fig. 2D illustrates an alternative cross-sectional view of the surgical retractor illustrated in Fig. 2A in an uncompressed-expanded configuration. Alternatively, if additional rigidity is needed, retractor 200 can be configured without stopping point 208 thereby permitting wire 210 to continue to circle around and within lumen of frame 202 as illustrated in Fig. 2D.

Membrane 204 provides a flexible surface against which delicate tissues and organs may contact without damaging such tissues and provides a barrier surface to prevent such tissues and organs from migrating from the retracted area and into the surgical site. Accordingly, unwanted tissues and organs are kept separate from the surgical site.

When retractor 200 is no longer needed, wire 210 may be pulled out from frame 202 thereby compressing retractor 200 rapidly (or as slowly as desired depending upon the amount of speed used to remove wire 210). After compression or partial compression, retractor 200 can be removed from the surgical site.

Retractor 200 is preferably sized so that it may be used in conjunction with laparoscopic surgery. It is contemplated that retractor 200 may be manufactured having a variety of sizes, such as small, medium, and large, so as to accommodate a variety of anatomical structures and locations. Other configurations, sizes, and dimensions are contemplated depending upon the surgical site in which retractor 200 may be deployed.

Frame 202 is preferably made from a flexible sleeve or tubing, including but not limited to, rubber, woven materials, fabrics, and polyester.

Membrane 204 is preferably made from a deformable material, including but not limited to, nylon, rubber, silicone, and polyethylene, although other materials are contemplated, including but not limited to, plastic, fabric, woven materials, and polymers. Membrane 204 is preferably about 1-5 mm thick, although other dimensions are contemplated depending upon the surgical site in which retractor 200 is to be deployed. It is contemplated that membrane 204 be configured sufficiently thin and flexible such that it is puncturable in the event the retained organs or tissues need to be accessed from the surgical site.

Wire 210 is preferably made from stainless steel or nitinol, although other materials are contemplated, including but not limited to, Teflon®-coated materials (Teflon® is available from DuPont, USA), polyvinyl chloride ("PVC"), and rigid polymers. The ticker wire is configured, the more rigid wire will become.

In some embodiments, wire 210 is preferably biased into a circular or ovoid shape, such that when directed into frame 202, wire 210 will assume a circular or ovoid shape (or other desired configuration). For example, in some embodiments wire 210 may be configured from a shape memory alloy, including but not limited to nitinol, and shape set to assume a linear shape when subj ected to about air temperature and a circular or ovoid shape when subjected to bodily temperatures.

Alternatively, in some embodiments, frame 202 may be preloaded with a deformable wire that is configured to assume a circular or ovoid shape (or other desired configuration) when subj ected to a temperature or range of temperatures. For example, frame 202 may be preloaded with a wire made from a shape memory alloy, such as nitinol or other materials. Wire may be shape set to assume a compressed ovoid-like shape when subjected to air temperature and then configured to assume an expanded circular or ovoid shape (or other desired configuration) upon being subjected to bodily temperatures.

Alternatively, in some embodiments, wire 210 may be configured from a bistable material, including but not limited to a flattened layered stainless steel material forming a flattened wire composite that is deformable into a circular-shaped wire, such as one that it is configured to assume a circular or ovoid shape when activated from a straight shape. Activation means may include snapping or bending wire causing it to assume a circular or ovoid shape (or other desired configuration).

Alternatively, in some embodiments, wire 210 may be configured from a bistable reel composite material. Suitable bistable reel composites are available from RolaTube^{™} Technology, LTD, Lymington, UK and others.

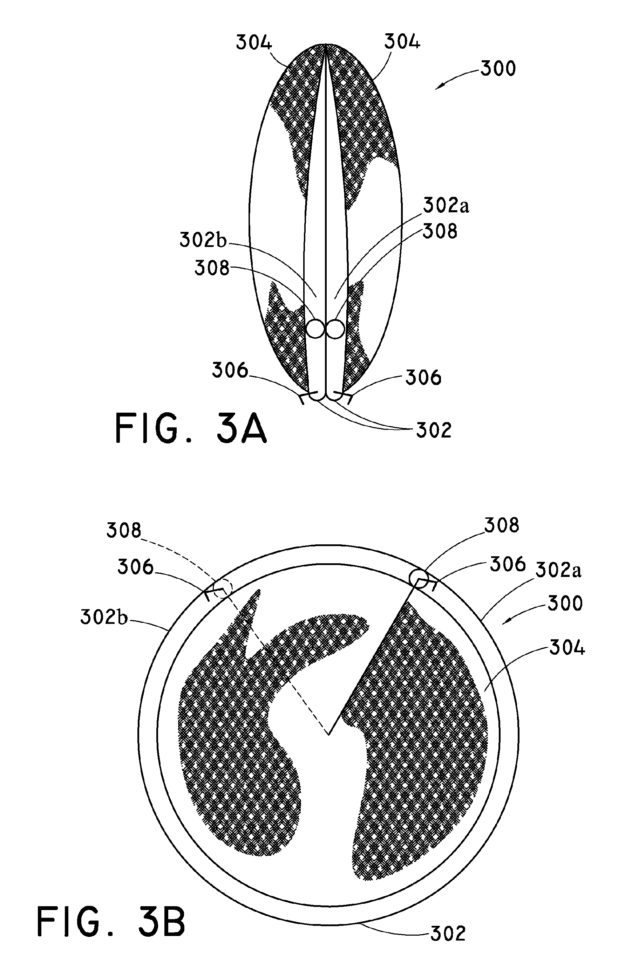

Fig. 3A illustrates a side view of surgical retractor 300 in a compressed configuration, and Fig. 3B illustrates a side view of surgical retractor 300 in an uncompressed-expanded configuration. Referring to Figs. 3A-3B, retractor 300 includes frame 302, membrane 304, hooks 306, and rings 308.

Retractor 300 is directed to hold back tissues and organs from a surgical site, including but not limited to, the bowel located in the abdomen, although retractor 300 can be used in other surgical and non-surgical applications. Retractor 300 is capable of assuming a compressed state (as illustrated in Fig. 3A) and an uncompressed-expanded state (as illustrated in Fig. 3B).

Retractor 300 is easy to deploy and position within an area of a patient, especially when used in conjunction with laparoscopic surgery and hand-assisted laparoscopic surgery. When configured in a compressed state (as illustrated in Fig. 3A), retractor 300 assumes a low profile making it easy to insert and position within a patient.

After being positioned, frame first portion 302a is gathered towards frame second portion 302b (or visa versa) and hooks 306 are engaged with rings 308 to thereby form a circular or ovoid shaped frame body 302 such that membrane 304 overlaps itself to prevent bowel, tissue, or other organs from escaping therethrough; other configurations are contemplated. Engagement means other than hooks 306 and rings 308 are contemplated. Accordingly, membrane 304 becomes sufficiently taut to provide a barrier to effectively retain organs and tissues from a surgical site. Frame 302 provides sufficient rigidity such that retractor 300 will not be pushed from the area in which it is located.

Membrane 304 provides a flexible surface against which delicate tissues and organs may contact without damaging such tissues and provides a barrier surface to prevent such tissues and organs from migrating from the retracted area and into the surgical site. Accordingly, unwanted tissues and organs are kept separate from the surgical site.

When retractor 300 is no longer needed, hooks 306 are disengaged from rings 308, and frame first portion 302a is directed away from frame second portion 302b (or visa versa) such that frame 302 partially splits causing retractor 300 to compress rapidly (or as slowly as desired by altering the speed with which frame first portion 302a and frame second portion 302b are directed away from each other). After compression or partial compression, retractor 300 can be removed from the surgical site.

Retractor 300 is preferably sized so that it may be used in conjunction with laparoscopic surgery. It is contemplated that retractor 300 may be manufactured having a variety of sizes, such as small, medium, and large, so as to accommodate a variety of anatomical structures and locations. Other configurations, sizes, and dimensions are contemplated depending upon the surgical site in which retractor 300 may be deployed.

Frame 302 is preferably made from a rubber, polymer, a polymer having a wire embedded therein, or other flexible material.

Membrane 304 is preferably made from a deformable material, including but not limited to, nylon, rubber, silicone, and polyethylene, although other materials are contemplated, including but not limited to, plastic, fabric, woven materials, and polymers. Membrane 304 is preferably about 1-5 mm thick, although other dimensions are contemplated depending upon the surgical site in which retractor 300 is to be deployed. It is contemplated that membrane 304 be configured sufficiently thin and flexible such that it is puncturable in the event the retained organs or tissues need to be accessed from the surgical site.

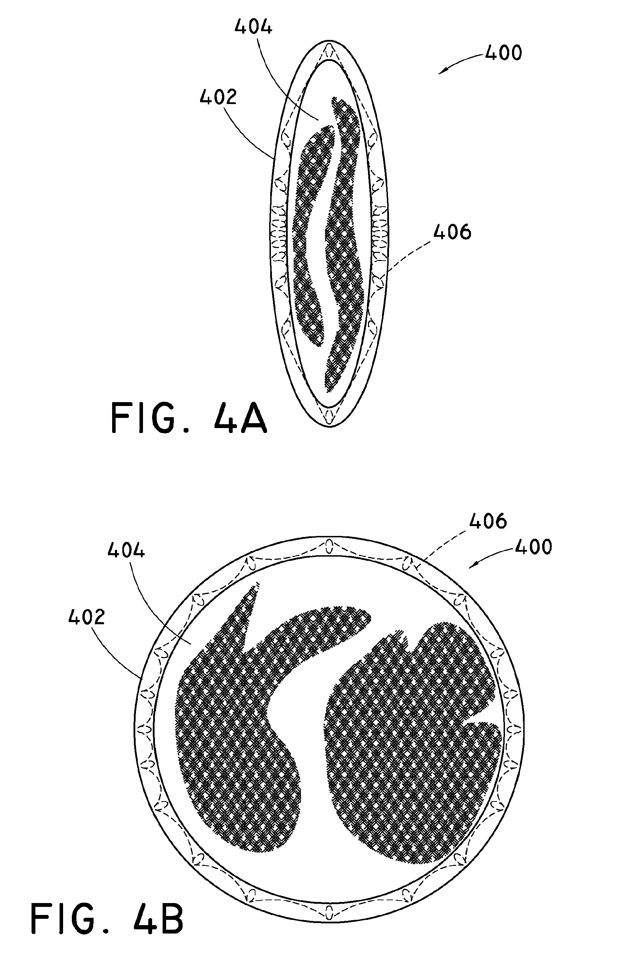

Fig. 4A illustrates a cross-sectional view of surgical retractor 400 in a compressed configuration, and Fig. 4B illustrates a cross-sectional view of surgical retractor 400 illustrated in Fig. 4A in an uncompressed-expanded configuration. Referring to Figs. 4A-4B, retractor 400 includes frame 402, membrane 404, and spring 406 disposed within a lumen of frame 402.

Retractor 400 is directed to hold back tissues and organs from a surgical site, including but not limited to, the bowel located in the abdomen, although retractor 400 can be used in other surgical and non-surgical applications. Retractor 400 is capable of assuming a compressed state (as illustrated in Fig. 4A) and an uncompressed-expanded state (as illustrated in Fig. 4B).

Retractor 400 is easy to deploy and position within an area of a patient, especially when used in conjunction with laparoscopic surgery and hand-assisted laparoscopic surgery. When configured in a compressed state (as illustrated in Fig. 4A), retractor 400 assumes a low profile making it easy to insert and position within a patient.

Retractor 400 is preloaded with spring 406. Retractor 400 is held in a compressed orientation for positioning, such as by applying a sufficient inwardly-directed force (such as by squeezing the outer surface of frame 402). Applying a sufficient inwardly-directed force to the outer surface of frame 402 causes spring 406, disposed within frame 402, to compress, thereby compressing retractor 400.

After being positioned, the sufficient inwardly-directed force is released to thereby permit frame 402 to be expanded by spring 406, which assumes its biased circular or ovoid shape (or other desired configuration). Accordingly, when uncompressed, frame 402 expands causing membrane 404 to become sufficiently taut to provide a barrier to effectively retain organs and tissues from a surgical site. The combination of spring 406 and frame 402 provides sufficient rigidity such that retractor 400 will not be pushed from the area in which it is located.

Membrane 404 provides a flexible surface against which delicate tissues and organs may contact without damaging such tissues and provides a barrier surface to prevent such tissues and organs from migrating from the retracted area and into the surgical site. Accordingly, unwanted tissues and organs are kept separate from the surgical site.

When retractor 400 is no longer needed, frame 402 and spring 406 may be cut to cause spring 406 to assume a straightened shape, thereby permitting easy removal of retractor 400.

Alternatively, when retractor 400 is no longer needed, a sufficient inwardly-directed force (such as by squeezing frame 402) may be applied to the outer surface of frame 402 thereby causing spring 406 to compress thereby compressing retractor 400 rapidly (or as slowly as desired by altering the speed at which the sufficient inwardly-directed compressive forces are applied to frame 402). After compression or partial compression, retractor 400 can be removed from the surgical site.

Retractor 400 is preferably sized so that it may be used in conjunction with laparoscopic surgery. It is contemplated that retractor 400 may be manufactured having a variety of sizes, such as small, medium, and large, so as to accommodate a variety of anatomical structures and locations. Other configurations, sizes, and dimensions are contemplated depending upon the surgical site in which retractor 400 may be deployed.

Frame 402 is preferably made from a rubber, polymer, silicone, fabric, or other flexible material which will sufficiently protect the body from spring 406, although other materials are contemplated.

Membrane 404 is preferably made from a deformable material, including but not limited to, nylon, rubber, silicone, and polyethylene, although other materials are contemplated, including but not limited to, plastic, fabric, woven materials, and polymers. Membrane 404 is preferably about 1-5 mm thick, although other dimensions are contemplated depending upon the surgical site in which retractor 400 is to be deployed. It is contemplated that membrane 404 be configured sufficiently thin and flexible such that it is puncturable in the event the retained organs or tissues need to be accessed from the surgical site.

Spring 406 is preferably made from stainless steel or nitinol, although other materials are contemplated. Spring 406 is preferably configured to exert an outward force.

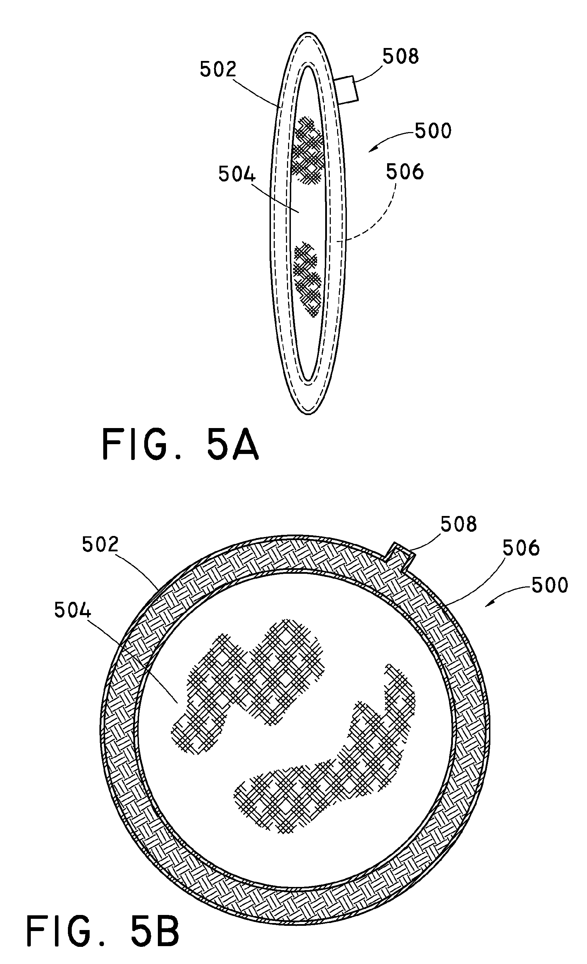

Fig. 5A illustrates a cross-sectional view of surgical retractor 500 in a compressed configuration, and Fig. 5B illustrates a cross-sectional view of surgical retractor 500 in an uncompressed-expanded configuration. Referring to Figs. 5A-5B, retractor 500 includes frame 502, membrane 504, and state changing material 506 disposed within a lumen of frame 502.

Retractor 500 is directed to hold back tissues and organs from a surgical site, including but not limited to, the bowel located in the abdomen, although retractor 500 can be used in other surgical and non-surgical applications. Retractor 500 is capable of assuming a compressed state (as illustrated in Fig. 5A) and an uncompressed-expanded state (as illustrated in Fig. 5B).

Retractor 500 is easy to deploy and position within an area of a patient, especially when used in conjunction with laparoscopic surgery and hand-assisted laparoscopic surgery. When configured in a compressed state (as illustrated in Fig. 5A), retractor 500 assumes a low profile making it easy to insert and position within a patient.

After being positioned within a surgical site, state changing material 506 is activated to cause frame 502 to become rigid and assume a circular or ovoid shape (or any other desired shape), thereby causing membrane 504 to become sufficiently taut to retain tissues and organs. Frame 502 provides sufficient rigidity such that retractor 500 will not be pushed from the area in which it is located.

Membrane 504 provides a flexible surface against which delicate tissues and organs may contact without damaging such tissues and provides a barrier surface to prevent such tissues and organs from migrating from the retracted area and into the surgical site. Accordingly, unwanted tissues and organs are kept separate from the surgical site.

When retractor 500 is no longer needed, retractor 500 can be removed from the surgical site.

Retractor 500 is preferably sized so that it may be used in conjunction with laparoscopic surgery. It is contemplated that retractor 500 may be manufactured having a variety of sizes, such as small, medium, and large, so as to accommodate a variety of anatomical structures and locations. Other configurations, sizes, and dimensions are contemplated depending upon the surgical site in which retractor 500 may be deployed.

Frame 502 is preferably made from a high-pressure, non-compliant, fluid impermeable material, including but not limited to, nylon, polyethylene terephthalate ("PET"), silicone, polyesters, and polyurethanes, although other materials are contemplated. Alternatively, frame 500 may be configured having no center portion such that membrane 504 is not needed. Accordingly, frame 500 may be configured so as to comprise a fillable apparatus comprising the entirety of the apparatus, such that the frame itself also serves as the barrier.

Membrane 504 is preferably made from a deformable material, including but not limited to, nylon, rubber, silicone, and polyethylene, although other materials are contemplated, including but not limited to, plastic, fabric, woven materials, and polymers. Membrane 504 is preferably about 1-5 mm thick, although other dimensions are contemplated depending upon the surgical site in which retractor 500 is to be deployed. It is contemplated that membrane 504 be configured sufficiently thin and flexible such that it is puncturable in the event the retained organs or tissues need to be accessed from the surgical site.

State changing material 506 is preferably made from foam, such as a polyurethane foam, although other materials are contemplated. For example, if foam is used, foam is preloaded into frame 502, and the air is removed from frame 502, such that frame 502 assumes a compressed state. After being positioned within a patient, air is directed into port 508, thereby causing foam to assume an expanded configuration and expand retractor 500. When retractor 500 is longer needed, air is vacuumed out from frame 502, thereby causing retractor 500 to assume a compressed state. Port 508 is preferably equipped with a one-way valve such that foam and air will not leak therefrom if the inflation tube (no shown) is decoupled from port 508.

Use of other state changing materials is contemplated. For example, state changing material 506 may comprise a super-cooled liquid, such as sodium acetate. For example, if sodium acetate is used, frame 502 is inserted into patient in a compressed state. Thereafter, sodium acetate is directed through port 508 and into frame 502. Thereafter, an activation seed is directed through port 508 causing sodium acetate to harden. When retractor 500 is longer needed, hardened sodium acetate is broken apart within frame 502, thereby causing retractor 500 to assume a compressed state while retaining sodium acetate debris within frame 502. Alternatively, frame may have a portion thereof configured for cutting, such that frame may be cut without having any portion of sodium acetate escape therefrom, and retractor 500 can then be removed from patient. Port 508 is preferably equipped with a one-way valve such that the sodium acetate and activation seed will not leak therefrom if the inflation tube (no shown) is decoupled from port 508.

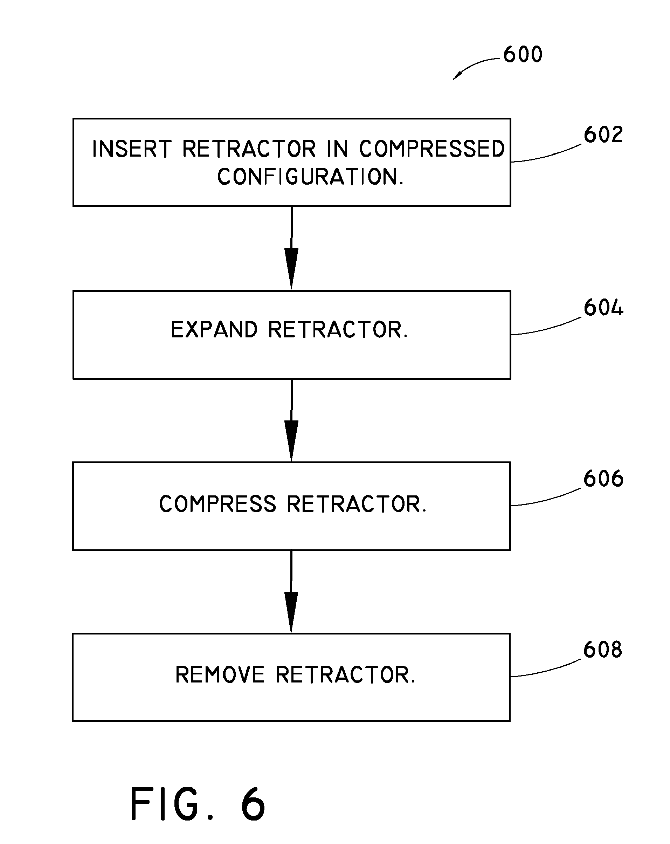

Fig. 6 illustrates a method for deploying a retractor, including but not limited to, those illustrated in Figs. 1A-5B. At block 602, a retractor configurable into a compressed configuration is inserted into a patient and directed to a surgical site. There, the retractor is placed between the surgical site and the area of tissues and/or organs to be retracted.

At block 604, the retractor is expanded. Means for configuring the retractor into an uncompressed-expanded configuration, include but are not limited to those illustrated in Figs. 1A-5B, such as: inflating the frame with a fluid, inserting a wire into the frame, activating a wire disposed within the frame, subj ecting the frame to a bodily temperature such that a deformable wire disposed within the frame assumes a circular or ovoid shape, attaching a frame first portion to a frame second portion, releasing a sufficient inwardly-directed force on an outer surface of the frame, or activating a state changing material disposed within the frame.

At block 606, after the retractor is no longer needed, the retractor is compressed. Means for configuring the retractor into a compressed configuration, include but are not limited to those illustrated in Figs. 1A-5B, such as: releasing a fluid from the frame, removing a wire from the frame, deactivating a wire disposed within the frame, subjecting the frame to a temperature such that a deformable wire disposed within the frame assumes a compressed configuration; disengaging a frame first portion from a frame second portion, applying a sufficient inwardly-directed force to an outer surface of the frame, or deactivating a state changing material disposed within the frame.

At block 608, the retractor is removed.

The retractors illustrated and equivalents thereto may be configured to assist with numerous surgical procedures, including but not limited to, laparoscopic or open sigmoid colectomy, ileocolic resection, hysterectomy, pelvic floor repair or resection fixation, or repair of the rectum or bladder. Retractors illustrated and equivalents thereto may be inserted into the abdominal cavity of a patient undergoing surgery via a SILS^{™} port access (available from Covidien, Mansfield, MA), hand assisted laparoscopic surgery access port, laparoscopic access device, or through an abdominal wall incision.

Once deployed, retractors illustrated or equivalents thereto generally assume a circular or ovoid shape (or other desired configuration) within the peritoneal cavity and are further urged into a convex form by pressure from the patient's lateral abdominal wall. During surgery, retractors illustrated and equivalents thereto are held in place by means of anterior-posterior pressure exerted on frames illustrated and equivalents thereto by the anterior abdominal wall and posterior retroperitoneal structures. Retractors illustrated and equivalents thereto are also held in place by lateral pressure exerted on retractors illustrated and equivalents thereto by the lateral abdominal wall. Accordingly, retractors illustrated and equivalents thereto are self-retaining. Having become sufficiently "wedged" into the abdominal cavity, membranes illustrated and equivalents thereto and/or frames illustrated and equivalents thereto exert axial pressure on the abdominal viscera thereby providing a barrier and preventing bowel loops (and/or other organs and/or tissues) from entering the surgical site.

In some embodiments, a retractor may be disposable. In some embodiments, a retractor may be reusable. It is also contemplated that in some embodiments, a retractor may be configured from one or more materials and one or more components parts.

From the foregoing, the discovery of apparatuses and methods of use of surgical retractors solves the problems presently posed through use of the Trendelenberg position, present graspers, and present retractors. It can be seen that the embodiments illustrated and equivalents thereto as well as the methods of manufacturer may utilize machines or other resources, such as human beings, thereby reducing the time, labor, and resources required to manufacturer the embodiments. Indeed, the discovery is not limited to the embodiments illustrated herein, and the principles and methods illustrated herein can be applied and configured to any retractors and equivalents.

Those of skill in the art will appreciate that embodiments not expressly illustrated herein may be practiced within the scope of the present discovery, including that features described herein for different embodiments may be combined with each other and/or with currently-known or future-developed technologies while remaining within the scope of the claims presented here. It is therefore intended that the foregoing detailed description be regarded as illustrative rather than limiting. It is understood that the following claims, including all equivalents, are intended to define the spirit and scope of this discovery. Furthermore, the advantages described above are not necessarily the only advantages of the discovery, and it is not necessarily expected that all of the described advantages will be achieved with every embodiment of the discovery.

The following numbered clauses set out specific embodiments that may be useful in understanding the present invention:

### CLAUSES

1. A surgical retractor comprising:
   a frame; and
   a membrane, wherein a perimeter of the membrane is at least partially bounded by the frame;
   means for configuring the surgical retractor between a compressed configuration and an uncompressed-expanded configuration, wherein the surgical retractor comprises a width when in the compressed configuration that is less than the width of the surgical retractor when in the uncompressed-expanded configuration; and
   wherein when in an uncompressed-expanded configuration, the membrane is sufficiently taut.
2. The surgical retractor of clause 1, wherein the means for configuring the surgical retractor into an uncompressed-expanded configuration comprises at least one of: inflating the frame with a fluid, inserting a wire into the frame, activating a wire disposed within the frame, subjecting the frame to a bodily temperature such that a deformable wire disposed within the frame assumes a circular or ovoid shape, attaching a frame first portion to a frame second portion, releasing a sufficient inwardly-directed force on an outer surface of the frame, or activating a state changing material disposed within the frame.
3. The surgical retractor of clause 2, wherein the means for configuring the surgical retractor into an uncompressed-expanded configuration comprises inflating the frame with a fluid.
4. The surgical retractor of clause 2, wherein the means for configuring the surgical retractor into an uncompressed-expanded configuration comprises inserting a wire into the frame.
5. The surgical retractor of clause 2, wherein the means for configuring the surgical retractor into an uncompressed-expanded configuration comprises activating a wire disposed within the frame.
6. The surgical retractor of clause 2, wherein the means for configuring the surgical retractor into an uncompressed-expanded configuration comprises subjecting the frame to a bodily temperature such that a deformable wire disposed within the frame assumes a circular or ovoid shape.
7. The surgical retractor of clause 2, wherein the means for configuring the surgical retractor into an uncompressed-expanded configuration comprises attaching a frame first portion to a frame second portion.
8. The surgical retractor of clause 2, wherein the means for configuring the surgical retractor into an uncompressed-expanded configuration comprises releasing a sufficient inwardly-directed force on an outer surface of the frame.
9. The surgical retractor of clause 2, wherein the means for configuring the surgical retractor into an uncompressed-expanded configuration comprises activating a state changing material disposed within the frame.
10. The surgical retractor of clause 2, wherein the means for configuring the surgical retractor into a compressed configuration comprises releasing a fluid from the frame.
11. The surgical retractor of clause 2, wherein the means for configuring the surgical retractor into a compressed configuration comprises removing a wire from the frame.
12. The surgical retractor of clause 2, wherein the means for configuring the surgical retractor into a compressed configuration comprises deactivating a wire disposed within the frame.
13. The surgical retractor of clause 2, wherein the means for configuring the surgical retractor into a compressed configuration comprises subjecting the frame to a temperature such that a deformable wire disposed within the frame assumes a compressed configuration.
14. The surgical retractor of clause 2, wherein the means for configuring the surgical retractor into a compressed configuration comprises disengaging a frame first portion from a frame second portion.
15. The surgical retractor of clause 2, wherein the means for configuring the surgical retractor into a compressed configuration comprises applying a sufficient inwardly-directed force to an outer surface of the frame.
16. The surgical retractor of clause 2, wherein the means for configuring the surgical retractor into a compressed configuration comprises deactivating a state changing material disposed within the frame.

## Claims

1. A surgical retractor comprising:
a frame; and
a membrane, wherein a perimeter of the membrane is at least partially bounded by the frame;
means for configuring the surgical retractor between a compressed configuration and an uncompressed-expanded configuration, wherein the surgical retractor comprises a width when in the compressed configuration that is less than the width of the surgical retractor when in the uncompressed-expanded configuration; and
wherein when in an uncompressed-expanded configuration, the membrane is sufficiently taut.

2. The surgical retractor of any preceding claim, wherein the means for configuring the surgical retractor into an uncompressed-expanded configuration comprises inflating the frame with a fluid.

3. The surgical retractor of any preceding claim, wherein the means for configuring the surgical retractor into an uncompressed-expanded configuration comprises inserting a wire into the frame.

4. The surgical retractor of any preceding claim, wherein the means for configuring the surgical retractor into an uncompressed-expanded configuration comprises activating a wire disposed within the frame.

5. The surgical retractor of any preceding claim, wherein the means for configuring the surgical retractor into an uncompressed-expanded configuration comprises subjecting the frame to a bodily temperature such that a deformable wire disposed within the frame assumes a circular or ovoid shape.

6. The surgical retractor of any preceding claim, wherein the means for configuring the surgical retractor into an uncompressed-expanded configuration comprises attaching a frame first portion to a frame second portion.

7. The surgical retractor of any preceding claim, wherein the means for configuring the surgical retractor into an uncompressed-expanded configuration comprises releasing a sufficient inwardly-directed force on an outer surface of the frame.

8. The surgical retractor of any preceding claim, wherein the means for configuring the surgical retractor into an uncompressed-expanded configuration comprises activating a state changing material disposed within the frame.

9. The surgical retractor of any preceding claim, wherein the means for configuring the surgical retractor into a compressed configuration comprises releasing a fluid from the frame.

10. The surgical retractor of any preceding claim, wherein the means for configuring the surgical retractor into a compressed configuration comprises removing a wire from the frame.

11. The surgical retractor of any preceding claim, wherein the means for configuring the surgical retractor into a compressed configuration comprises deactivating a wire disposed within the frame.

12. The surgical retractor of any preceding claim, wherein the means for configuring the surgical retractor into a compressed configuration comprises subjecting the frame to a temperature such that a deformable wire disposed within the frame assumes a compressed configuration.

13. The surgical retractor of any preceding claim, wherein the means for configuring the surgical retractor into a compressed configuration comprises disengaging a frame first portion from a frame second portion.

14. The surgical retractor of any preceding claim, wherein the means for configuring the surgical retractor into a compressed configuration comprises applying a sufficient inwardly-directed force to an outer surface of the frame.

15. The surgical retractor of any preceding claim, wherein the means for configuring the surgical retractor into a compressed configuration comprises deactivating a state changing material disposed within the frame.
